# EUROPEAN PATENT SPECIFICATION

(11) **EP 4 355 386 B1**
(45) Date of publication and mention of the grant of the patent: **06.08.2025**
(21) Application number: 22735611.0
(22) Date of filing: 14.06.2022
(51) Int. Cl.: A61M 5/142, A61M 5/158

(54) **APPLICATOR FOR INFUSION DEVICES**
APPLIKATOR FÜR INFUSIONSVORRICHTUNGEN
APPLICATEUR POUR DISPOSITIFS DE PERFUSION

(30) Priority: 16.06.2021 IT 202100015722
(43) Date of publication of application: 24.04.2024
(73) Proprietor: Theras Lifetech S.r.l., 43039 Salsomaggiore Terme (PR) (IT)
(72) Inventor: BOBBI, Maria, 43015 Noceto (Parma) (IT); FERRARI, Federico, 43039 Salsomaggiore Terme (Parma) (IT); ZANARDINI, Francesco, 25057 Sale Marasino (Brescia) (IT); CENTOLA, Matteo, 40137 Bologna (IT)
(74) Representative: Persi, Patrizia
(86) International application number: PCT/IB2022/055490
(87) International publication number: WO 2022/264025

(56) References cited:
- WO-A1-2015/168219
- US-A1- 2007 293 826
- US-A1- 2017 020 458

## Description

This invention relates to an applicator for infusion devices.

Moreover, the invention relates to an infusion system and a method for using the infusion system.

Infusion devices are devices for releasing medicines in the body of a patient or of a user.

Such devices comprise catheters for delivering fluid which include a cannula suitable for insertion into a cavity of the body of a patient. In particular, the cannula allows the medicine to flow and hence to deliver the medicine to the patient.

Such devices are provided with a base portion and a cap portion which define a body that partly contains the catheter. The cannula protrudes, in fact, from the base portion in such a way as to allow it to be inserted into the body of the patient.

The base portion comprises a sticking plaster to allow the device to be attached to the patient. The sticking plaster comprises a peelable sheet and an adhesive component. In particular, the peelable sheet is removable in such a way as to make accessible the adhesive component intended to adhere to the skin of the patient.

The cap portion is fixed to an insertion needle which is designed to penetrate the skin during a first insertion of the cannula. The insertion needle is, in fact, of a size such that it can be housed in the catheter and comes out of the cannula in such a way that it can reach and penetrate the skin so that the subsequent first insertion of the cannula can be made possible.

However, these devices may be difficult to insert for a non-expert user who must apply the infusion device on him/herself.

In particular, the fear of the pain or the inexperience might lead to the user inserting the device incorrectly.

For this reason, applicators are known for the above-mentioned infusion devices which are generally defined by snap-on mechanisms which allow the application of the device after the pressing of a button or other similar actuating means.

Patent documents US2017020458A1, US2007293826A1 and WO2015168219A1 describe further types of devices.

US2017020458A1 describes an insertion device comprising a grip positioned in a position proximal to the base. According to a first configuration, the needle is located in a position spaced from the adhesive layer. The device is provided with an inner guide comprising a carriage. In an insertion position the handle is lowered by the pushing action of the spring. A shuttle supports the needle retaining device. The internal guide, the shuttle and the base are connected to each other by the flaps and the fingers. The shuttle and the guide are axially mobile relative to the base. When the device is moved towards the skin of the user, the needle is inserted into the skin. When the flanges of the base engage the fingers after the insertion movement, the fingers disengage from the edge of the guide, thus withdrawing the needle.

US2007293826A1 describes a manual feeding injection device which self-administers a painless injection. The injection device comprises a housing having an outer body, a carriage for a needle, means for retaining a liquid composition and a base. The carriage is configured for performing a movement along the central axis perpendicular to the base. The device is provided with a guide wall in which the carriage can move. A retaining heel is pushed towards the inside from an opening in the guide wall. The withdrawal spring is compressed between the lower part of the carriage and the base. When the carriage passes to the completely inserted position, it passes by the heel which momentarily leaves the opening and then re-inserts. The heel forms part of a release arm which, when pressed, allows the needle to be withdrawn.

Patent WO2015168219A1 describes a control device, which can be used in an apparatus for administering medicines, comprising a housing, a pushing mechanism mounted to move with respect to the housing, a supplier of force positioned between the housing and the pushing mechanism for moving the pushing mechanism with respect to the housing, and a coupling mechanism to selectively limit and allow the relative movement between the housing and the pushing mechanism. The dragging mechanism can be configured to push against a tank of the apparatus for transdermal administration of the drug. The coupling mechanism can include flexible arms which extend between the opposite ends of the coupling mechanism, and the coupling mechanism can be configured in such a way that the arms release the pushing mechanism in response to the opposite ends which are forced towards each other.

Disadvantageously, the prior art applicators may suffer from problems such as, for example, the unwanted removal of the infusion device once the skin of the patient has been penetrated.

A further drawback may lie in the non-optimum adhesion of the sticking plaster which, during use or immediately after application of the infusion device, could yield and detach from the skin of the user, resulting in an undesired total or partial removal of the device. If the removal is partial and possibly imperceptible to the user it is also possible that there is a non-optimum dispensing of the fluid which would therefore result in a potentially ineffective treatment.

The technical purpose of the invention is therefore to provide an applicator for infusion devices, an infusion system and a method for using the infusion system which is/are able to overcome the drawbacks of the prior art.

The aim of the invention is therefore to provide an applicator for infusion devices, an infusion system and a method for using the infusion system which allow(s) an optimum adhesion of the infusion device to be obtained.

A further aim of the invention is to provide an applicator for infusion devices, an infusion system and a method for using the infusion system which is/are easy to use also for a non-expert user.

The technical purpose indicated and the aims specified are substantially achieved by an applicator for infusion devices, an infusion system and a method for using the infusion system comprising the technical features described in one or more of the appended claims. The dependent claims correspond to possible embodiments of the invention.

In particular, the technical purpose indicated and the aims specified are substantially achieved by an applicator for infusion devices comprising a main body comprising an access opening and an upper opening coaxial with each other and defined respectively in a lower portion and an upper portion of the main body, a central body positioned inside the main body and comprising a containment seat for the infusion device facing towards the access opening for housing the infusion device, a dragging element comprising a first portion, wherein a first end is connected or connectable to the central body and passes through the upper opening, and a second portion, which can be pressed by a user, positioned at a second end of the first portion, and actuating means connected or connectable to the central body.

The central body is movable inside the main body between a first configuration, wherein the central body is positioned in the lower portion of the main body and the first portion of the dragging element is positioned inside the main body, and a second configuration wherein the central body is positioned in the upper portion of the main body and the first portion of the dragging element is positioned outside the main body.

The central body is movable by pressure from the user from the first configuration to the second and the actuating means can be operated by a user to move said central body from the second to the first configuration for applying, in use, the infusion device, housed in the containment seat of the central body, to the skin of the user.

The second portion can be pressed, when the central body is in the first configuration after the application of the infusion device, in such a way as to further move the central body towards the access opening to promote a homogeneous adhesion of the sticking plaster of the infusion device to the skin.

Moreover, the technical purpose indicated and the aims specified are substantially achieved by an infusion system comprising an applicator described above and an infusion device comprising an infusion body, comprising a supporting base from which protrudes a cannula-shaped element of a catheter, and a removable closing element provided with a needle suitable for penetrating the skin of the body of the user. The supporting base comprises a sticking plaster to allow the device to be attached to the body of the patient.

The infusion device can be coupled by fitting into the containment seat of the central body of the applicator.

Moreover, the technical purpose indicated and the aims specified are substantially achieved by a method for using an above-mentioned system and comprising the steps of preparing an applicator, preparing an infusion device, coupling the infusion device by fitting in the containment seat of the central body, pressing the central body, to which the infusion device is coupled, inside the main body defining the second configuration, placing the applicator on the skin of a user, operating the actuating means by moving the central body to the first configuration in such a way as to apply the infusion device to the skin of the patient, further moving the central body towards the access opening, by pressing the second portion of the dragging element, to promote a homogeneous adhesion of the infusion device to the skin and moving said applicator away from the skin of the user.

Further features and advantages of the invention are more apparent in the non-limiting description which follows of a non-exclusive embodiment of an applicator for infusion devices, an infusion system and a method for using the infusion system.

The description is set out below with reference to the accompanying drawings which are provided solely for purposes of illustration without restricting the scope of the invention and in which:
- Figure 1 is a schematic exploded view of the applicator according to the invention;
- Figures 2A and 2B are perspective views of a system according to the invention;
- Figures 3A-3E schematically illustrate various steps in the use of the applicator according to Figures 1, 2A and 2B;
- Figure 4 is a schematic exploded view of a further embodiment of the applicator;
- Figures 5A-5E schematically illustrate various steps in the use of the applicator according to Figure 4.

With reference to the accompanying drawings, the numeral 1 denotes in its entirety an applicator for infusion devices 10 which, for simplicity of description, will hereafter be referred to as applicator 1.

The applicator 1 comprises a main body 2 defining a containment space and comprising an access opening 2a and an upper opening 2b. The access opening 2a and the upper opening 2b are coaxial with each other and are defined respectively in a lower portion 2c and an upper portion 2d of the main body 2. The terms "lower" and "upper" refer to the orientation of the applicator 1 as shown in the accompanying drawings. Moreover, the lower portion 2c and the upper portion 2d may be indicated both with reference to external and/or internal portions of the main body 2 and with reference to the applicator 1.

The main body 2 also comprises at least one lateral opening 2e. Preferably, the lateral opening 2e is defined in the upper portion 2d of the main body 2.

Preferably, and as shown in the accompanying drawings, the main body 2 may comprise two lateral openings 2e.

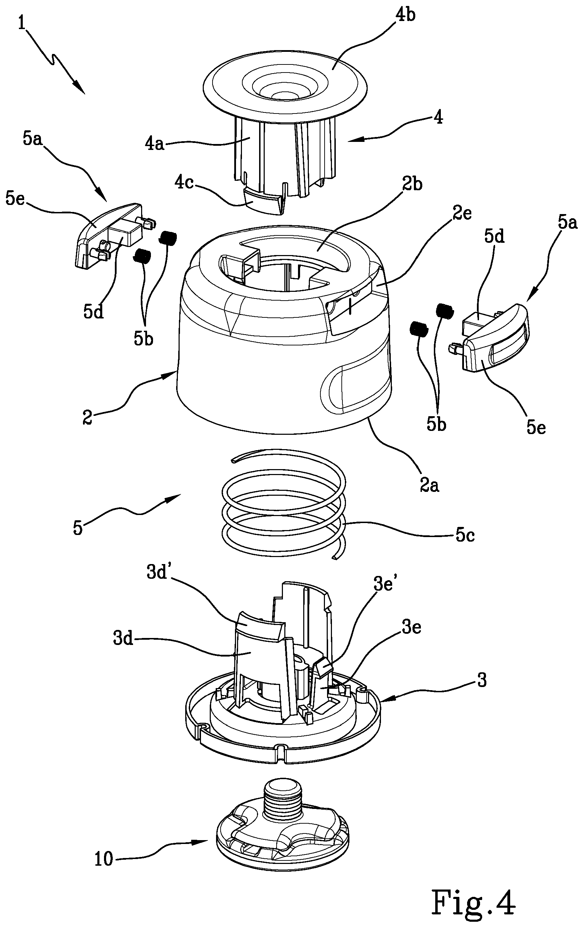

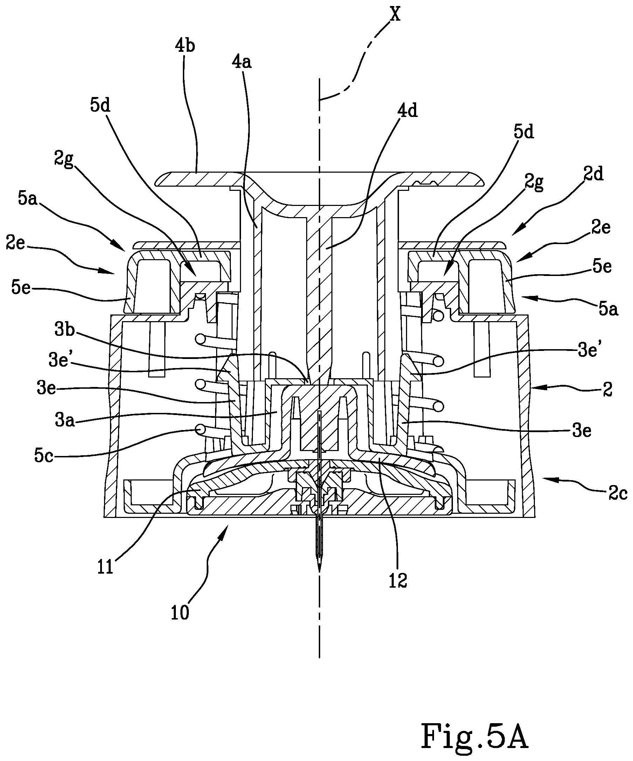

Preferably, with reference to a further embodiment illustrated in Figures 4 and 5A-5E, which will be described in more detail below, the main body 2 may comprise at least one guide 2g, in the upper portion 2d, having an end leading into the lateral opening 2e. Where, as shown in the accompanying drawings, the main body 2 comprises two lateral openings 2e, the main body 2 comprises two respective guides 2g.

Preferably, the guide 2g (or both the guides 2g) has a second end leading inside the main body 2.

Preferably, the main body 2 extends along a relative axis of extension "X". Preferably, and as shown in the accompanying drawings, the axis of extension "X" also defines the main axis of the applicator 1. For this reason, the access opening 2a and the upper opening 2b are coaxial with each other relative to the axis of extension "X".

Preferably, the main body 2 is substantially cylindrical in shape or in any case has a circular base section.

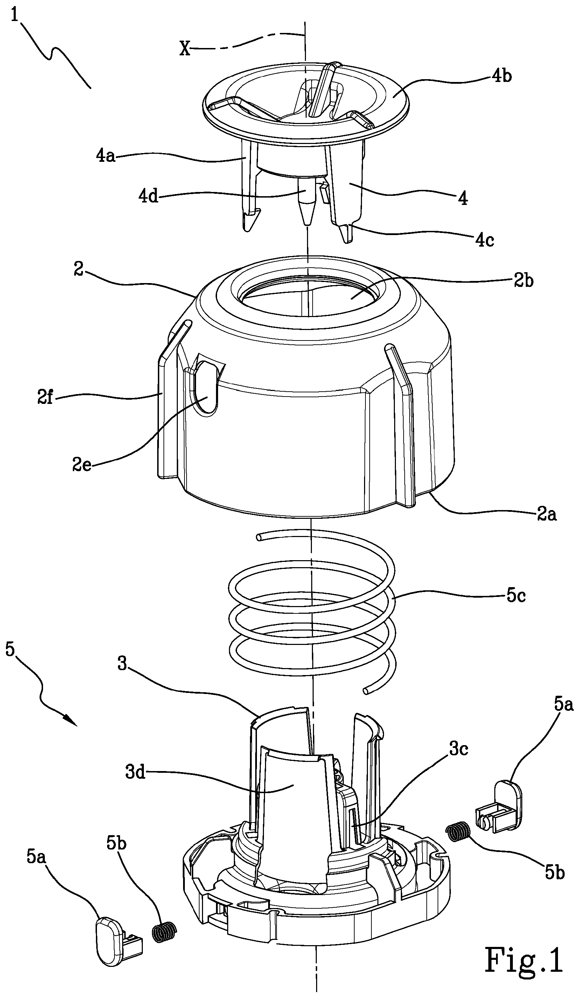

Preferably, the main body 2 has, on an outer surface of the main body 2, lateral flaps 2f defining an improved gripping surface, as shown for example in Figure 1.

The applicator 1 also comprises a central body 3 positioned inside the containment space and comprising a containment seat 3a for the infusion device 10. The containment seat 3a faces towards the access opening 2a. In other words, through the access opening 2a it is possible to introduce the infusion device 10 and couple it to the containment seat 3a.

Preferably, the containment seat 3a is shaped in such a way as to allow the coupling by the fitting of the infusion device 10.

Preferably, the containment seat 3a is shaped to match the infusion device 10.

Preferably, the containment seat 3a of the central body 3 also comprises a through hole 3b facing towards the upper opening 2b. In other words, the through hole 3b defines a passage between the containment seat 3a and the portion of containment space positioned above the central body 3.

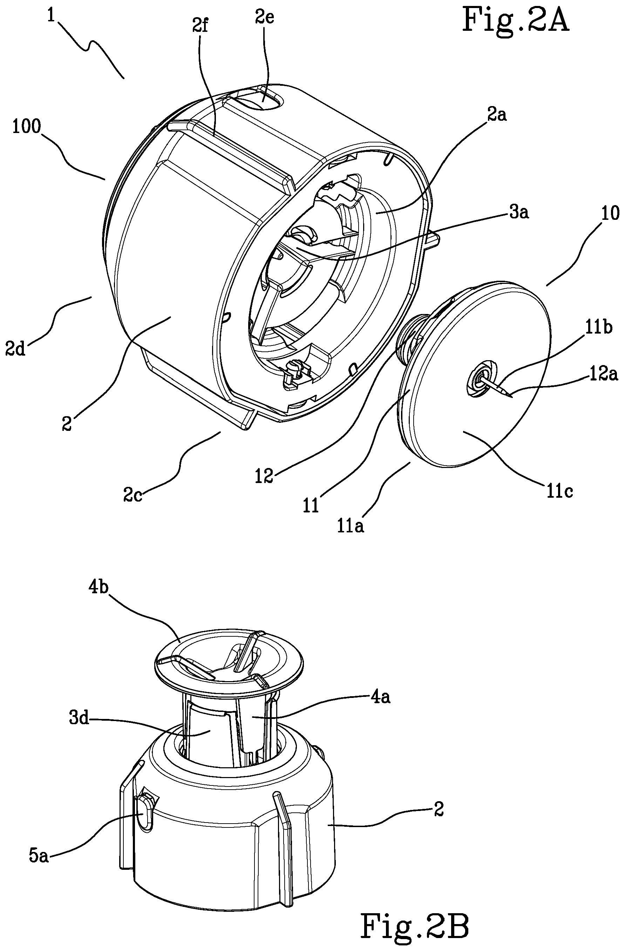

Preferably, the containment seat 3a of the central body 3 has a first portion directly facing the access opening having the shape substantially of a spherical cap and a second portion, positioned above the first portion, having a substantially cylindrical shape, as for example shown in the accompanying drawings (Figure 2A). The first portion having the shape substantially of a spherical cap is suitable for retaining an infusion body 11 of the infusion device 10 whilst the second portion having a substantially cylindrical shape is suitable for retaining at least one portion of a closing element 12 of the infusion device 10.

Other shapes of the containment seat 3a are possible depending on the shape of the respective infusion device 10.

Preferably, the central body 3 comprises a plurality of openings 3c defined on an upper edge of the central body 3. In other words, the openings 3c face towards the upper opening 2b.

Preferably, the central body 3 has a circular cross section.

The applicator 1 also comprises a dragging element 4 comprising a first portion 4a having a first end connected or connectable to the central body 3. The first portion 4a passes through the upper opening 2b. Moreover, the upper opening 2b defines a passage in which said first portion 4a can move to enter into or exit from the containment space.

The dragging element 4 also comprises a second portion 4b positioned at a second end of the first portion 4a. The second portion 4b defines a pressing surface which can be pressed by a user. The technical advantages of this pressing of the second portion 4b will be described in more detail below.

However, it should be noted that the dragging element 4 does not act as an actuating button of the applicator 1. In other words, the purpose of the dragging element 4 is not to actuate a mechanism for releasing the infusion device 10.

Preferably, the first portion 4a of the dragging element comprises a plurality of flaps 4c which can engage with a corresponding number of openings 3c of the central body 3c. In other words, the flaps 4c and the openings 3c define connecting means between the central body 3 and the dragging element 4.

Preferably, the dragging element 4 also comprises an elongate body 4d configured for, during a pressing of the second portion 4b, passing through the through hole 3b. In other words, the through hole 3b faces towards the dragging element 4. In a manner similar to that described above, the technical advantages of this feature will be described in more detail below. Preferably, and as for example shown in the accompanying drawings, the central body 3 comprises protruding portions 3d extending parallel to the first portion 4a of the dragging element 4 and configured to define an end stop of the dragging element 4 (in particular of the second portion 4b) when pressed.

Preferably, as shown for example in Figure 4, the protruding portions 3d are provided with hook-shaped ends 3d' to ensure the engaging between the central body 3 and the dragging element 4.

According to the further embodiment shown in Figures 4 and 5A-5E, the central body 3 also comprises retaining portions 3e extending substantially parallel to the first portion 4a of the dragging element 4 and having hook-shaped ends 3e'. The retaining portions 3e, in particular the ends 3e', are configured to be engaged inside the end of the guides 2g which leads inside the main body 2, as described in more detail below.

The applicator 1 also comprises actuating means 5 connected or connectable to the central body 3.

Preferably, the actuating means 5 comprise at least one button 5a movable towards or away from the central body 3.

Preferably, according to the embodiment wherein the central body 3 has a circular cross section, the button 5a is movable radially relative to the central body 3.

Preferably, with reference to the embodiment of Figures 1, 2A, 2B and 3A-3E, the button 5a may be connected or connectable to the central body 3 by a suitable elastic element, even more preferably by a spring 5b.

Preferably, with reference to the embodiment of Figures 4 and 5A-5E, the button 5a may comprise a sliding portion 5d and a pressable portion 5e. According to this embodiment, the sliding portion 5d of the button 5a is configured to slide in the guide 2g. Moreover, the button 5a is connected or connectable to the main body 2 by means of a suitable elastic element, even more preferably by at least one spring 5b, connecting the pressable portion 5e with the main body 2. In the accompanying drawings, the button 5a is connected to the main body 2 by two springs 5b.

Preferably, and as shown in the accompanying drawings, the actuating means 5 comprise two buttons 5a (and respective elastic elements) positioned in opposite lateral portions of the central body 3, that is to say, of the main body 2. According to this embodiment, the main body 2 comprises two lateral openings 2e positioned in opposite portions of the upper portion 2d of the main body 2.

Preferably, the actuating means 5 also comprise a loading spring 5c the ends of which are respectively connected to the central body 3 and to an inner perimeter edge of the upper opening 2b of the main body 2. With reference to Figures 5A-5E, the portion below the guide 2g defines a connecting portion, that is to say, retaining the upper end of the loading spring 5c.

With reference to the operation of the applicator 1, the central body 3 is movable inside the containment space (that is to say, inside the main body 2) between a first configuration and a second configuration (corresponding to a first and a second configuration of the applicator 1).

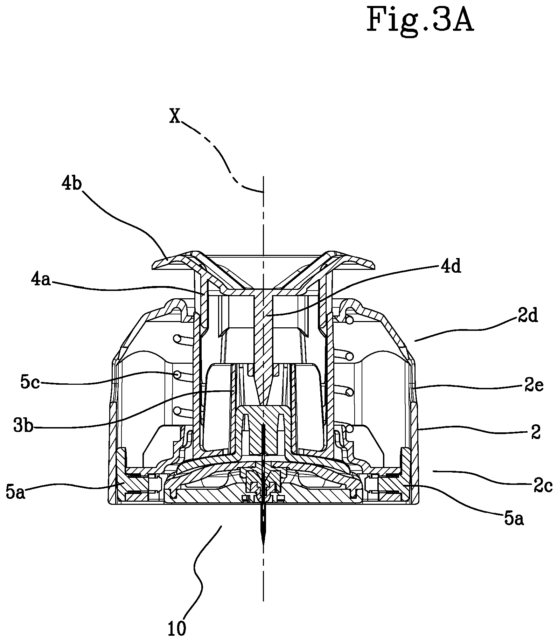

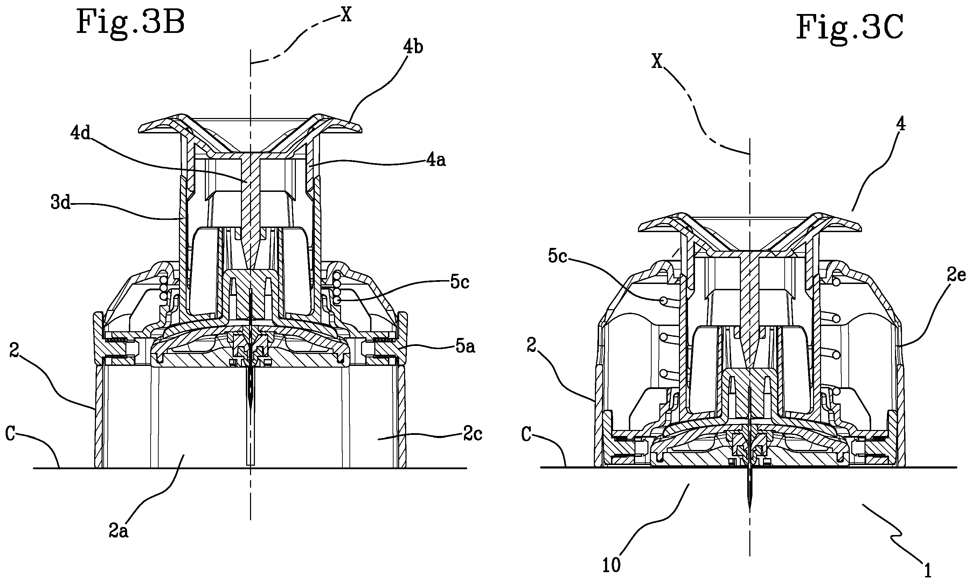

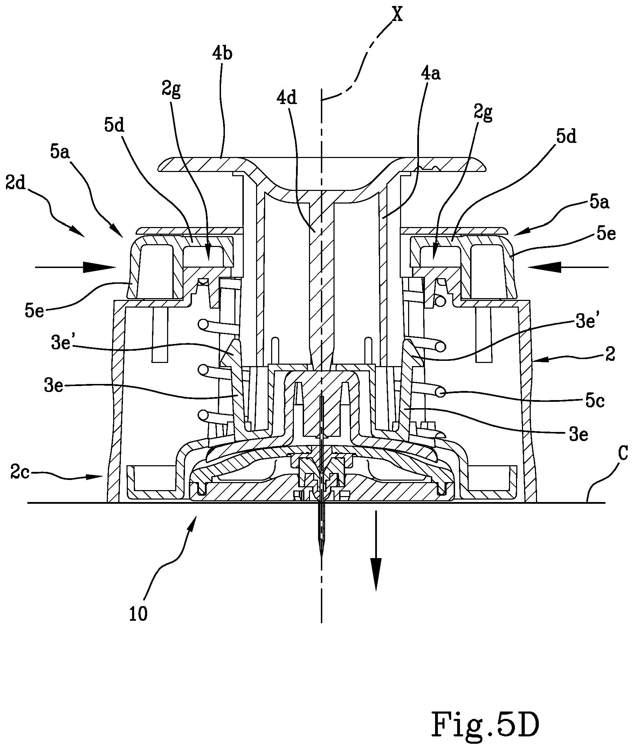

The first configuration of the applicator 1 coincides with a rest configuration of the applicator 1 (Figures 3A and 5A) or with a configuration after application of the infusion device 10 (Figures 3C and 5D).

In the first configuration the central body 3 is positioned in the lower portion 2c of the main body 2 and the first portion 4a of the dragging element 4 (and the second portion 4b alongside the upper opening 2b) and the actuating means 5 are internally positioned in the containment space.

With reference to the accompanying drawings, in the first configuration the loading spring 5c is in a rest position in which it extends between the inner perimeter edge of the upper opening 2b and the central body 3.

In Figure 3A, in this first configuration, the button 5a is placed in contact with an inner wall of the main body 2.

In Figure 5A, the retaining portion 3e is not engaged in the respective guide 2g.

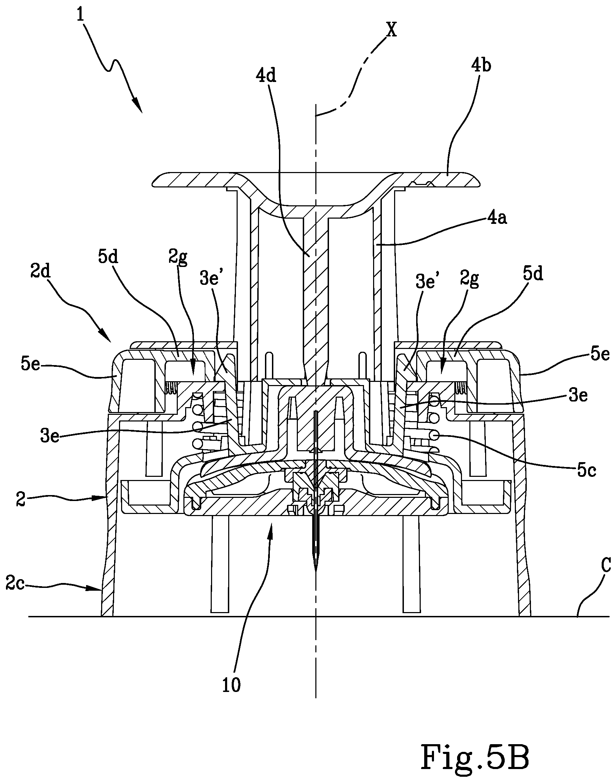

The second configuration of the applicator 1 coincides with a loading configuration of the applicator 1 (Figures 3B and 5B) wherein the infusion device 10 is inserted in the containment seat 3a to be subsequently applied to the skin "C" of the user.

In the second configuration of the applicator 1, the central body 3 is positioned in the upper portion 2d of the main body 2, the first portion 4a of the dragging element 4 is positioned outside the main body 2 (that is to say, protrudes from the upper opening 2b away from the main body 2) and the actuating means 5 are at least partly facing the at least one lateral opening 2e.

With reference to Figure 3B, in the second configuration of the applicator 1, the loading spring 5c is compressed between the inner perimeter edge and the central body 3 (raised in the upper portion 2d) and the button 5a protrudes from the respective lateral opening 2e in such a way as to lock the central body 3 in the upper portion 2d. In the accompanying drawings, both the buttons 5a protrude from the respective lateral openings 2e.

In other words, the elastic element (that is to say, the spring 5b) of each button 5a allows it to abut against the inner wall of the main body 2 (when the central body 3 is in the lower portion 2c) or push the button 5a outside the lateral openings 2e (when the central body 3 is in the upper portion 2d). In the second configuration, the buttons 5a and the respective elastic elements guarantee that a release of the loading spring 5c is prevented by locking the central body 3 and thus keeping the loading spring 5c compressed.

The central body 3 is movable by pressure by the user in such a way as to move the applicator 1 from the first configuration to the second configuration. In other words, once the infusion device 10 is inserted into the containment seat 3a, the user can push the central body 3 from the lower portion 2c to the upper portion 2d. In other words, by pressing, the central body 3 is moved in the upper portion 2d until the buttons 5a release, protruding from the lateral openings 2e. During this pressing movement the buttons 5a are able to slide on the inner wall of the main body 2.

With reference to Figure 5B and to the further embodiment, in the second configuration of the applicator 1, the loading spring 5c is compressed between the inner perimeter edge and the central body 3 (raised in the upper portion 2d) and the retaining portions 3e, in particular the ends 3e', are engaged in the respective end of the guides 2g in such a way as to lock the central body 3 in the upper portion 2d. In the accompanying drawings, both the buttons 5a (that is to say, the pressable portions 5e) protrude from the respective lateral openings 2e.

In other words, the elastic element (that is to say, the spring 5b) of each button 5a keeps the button (that is to say, its sliding portion 5d) away from the retaining portions 3e in such a way as not to accidentally release the central body 3.

In the second configuration, the retaining portions 3e guarantee that a release of the loading spring 5c is prevented by locking the central body 3 and thus keeping the loading spring 5c compressed.

By pressing, the central body 3 is moved into the upper portion 2d until the retaining portions 3e, in particular the hook-shaped ends 3e', release and engage in the ends of the guides 2g.

Once the applicator 1 has been loaded, the actuating means 5 may be operated by the user to move the applicator 1 from the second to the first configuration for applying the infusion device 10, housed in the containment seat 3a of the central body 3, to the skin "C" of the user.

With reference to the embodiment illustrated in the accompanying drawings, the buttons 5a can be pressed in such a way as to release the central body 3 and move the applicator 1 to the first configuration.

With reference to the embodiment of Figures 1, 2A, 2B and 3A-3E, by withdrawing the buttons 5a into the main body 2, the loading spring 5c is free to return to the uncompressed state releasing the central body 3 from the upper portion 2d to the lower portion 2c.

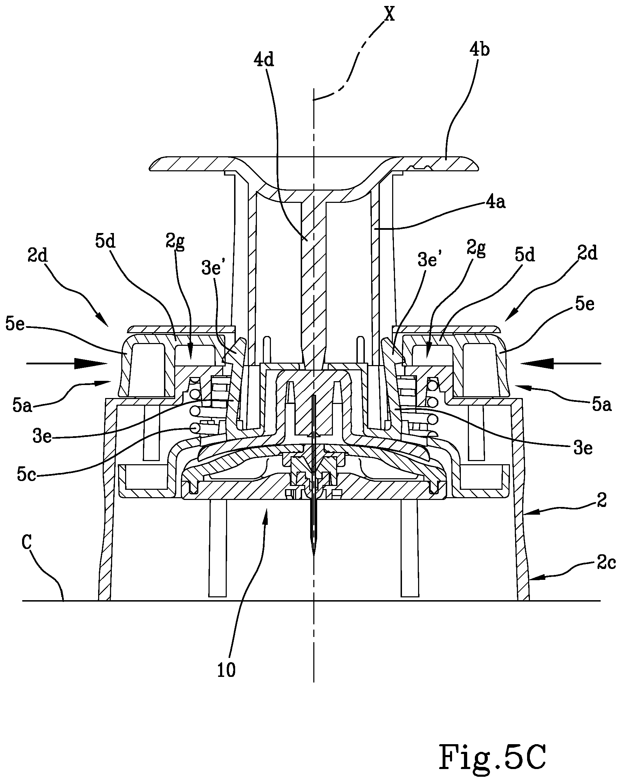

With reference, on the other hand, to the further embodiment of Figures 4 and 5A-5E, the pressing of the buttons 5a (that is to say, of the pressable portion 5e) allows the sliding portion 5d to slide inside the respective guide 2g to come into contact with the respective retaining portions 3e (Figure 5C), in particular with the ends 3e'. In other words, each sliding portion 5d is moved towards the respective end 3e' of the retaining portion 3e.

This sliding causes the sliding portions 5d to intercept the retaining portions 3e in such a way as to push them away from the guide 2g thereby freeing the loading spring 5c (Figure 5D). In other words, the pressing of the pressable portion 5e allows the sliding portion 5d to strike the retaining portion 3e engaged in the guide 2g, in particular the ends 3e', and disconnect it from the guide 2g, allowing the loading spring 5c to return to the uncompressed state releasing the central body 3 from the upper portion 2d to the lower portion 2c.

For this reason, both the buttons 5a must be pressed to allow the movement of the central body 3 from the upper portion 2d to the lower portion 2c. According to this embodiment, the pressing of a single button 5a would not allow the release of the central body 3. For this reason, the retaining portions 3e (that is to say, the ends 3e') must both be released from the respective guides 2g in order to release the central body 3.

For this reason, a user who wishes to apply the infusion device 10 on his/her skin "C" should move the loaded applicator 1 to his/her skin "C" in such a way that the access opening 2a is obstructed by the skin "C". After pressing the buttons 5a, the release of the loading spring 5c means that the central body 3 releases at the required speed so that the infusion device 10 can penetrate the skin "C" of the user and remains applied to it.

At this point, when the central body 3 is in the first configuration following the application of the infusion device 10, the dragging element 4 (that is to say, the second portion 4b) can be pressed by the user in such a way as to further move the central body 3 towards the access opening 2a for promoting a homogeneous adhesion of the infusion device 10 to the skin C.

In effect, thanks to this further movement, the first portion 4a pushes the central body 3 against the skin "C" of the user, guaranteeing that the infusion device 10 is able to uniformly adhere to the skin "C".

According to the embodiment in which the through hole 3b is present, the pressing of the second portion 4b allows the elongate body 4d to enter the through hole 3b in such a way as to directly push the infusion device 10 against the skin "C" of the patient. Moreover, the pressure may advantageously be used for separating at least the infusion body 11 of the infusion device 10 (which is the component which must necessarily remain applied to the skin "C" of the patient) from the containment seat 3a.

The invention also relates to an infusion system 100 comprising an applicator 1 according to one or more of the embodiments described above.

The infusion system 100 also comprises an infusion device 10 comprising an infusion body 11 and a closing element 12.

The infusion body 11 comprises a supporting base 11a from which protrudes a cannula-shaped element 11b of a catheter. The supporting base 11a comprises a sticking plaster 11c to allow the infusion device 10 to be attached to the body of the patient (that is to say, to the skin "C" of the patient).

Preferably, the sticking plaster 11c comprises a peelable sheet and an adhesive component. In particular, the peelable sheet is removable in such a way as to make accessible the adhesive component intended to adhere to the skin "C" of the user.

The closing element 12 is removably connected to the infusion body 11 and is provided with a needle 12a suitable for penetrating the skin "C" of the body of the user.

The infusion device 10 can be coupled by fitting into the containment seat 3a of the central body 3 of the applicator 1.

Preferably, the containment seat 3a is shaped to match the infusion device 10. Even more preferably, the containment seat 3a has a first portion directly facing the access opening 2a having the shape substantially of a spherical cap and a second portion, positioned above the first portion, having a substantially cylindrical shape. The two portions are respectively shaped to match the infusion body 11 and the closing element 12.

Following the pressing of the actuating means 5, the central body 3 is released in such a way that the needle 12a can penetrate the skin "C" of the user so as to allow insertion of the cannula-shaped element 11b through it. Moreover, during application of the infusion device 10, the sticking plaster 11c comes into contact with the user's skin "C", adhering to it.

During the subsequent pressing of the dragging element 4 it is therefore possible to make the adherence of the sticking plaster 11c as uniform as possible, thereby improving the application of the infusion device 10 to the skin "C" of the user.

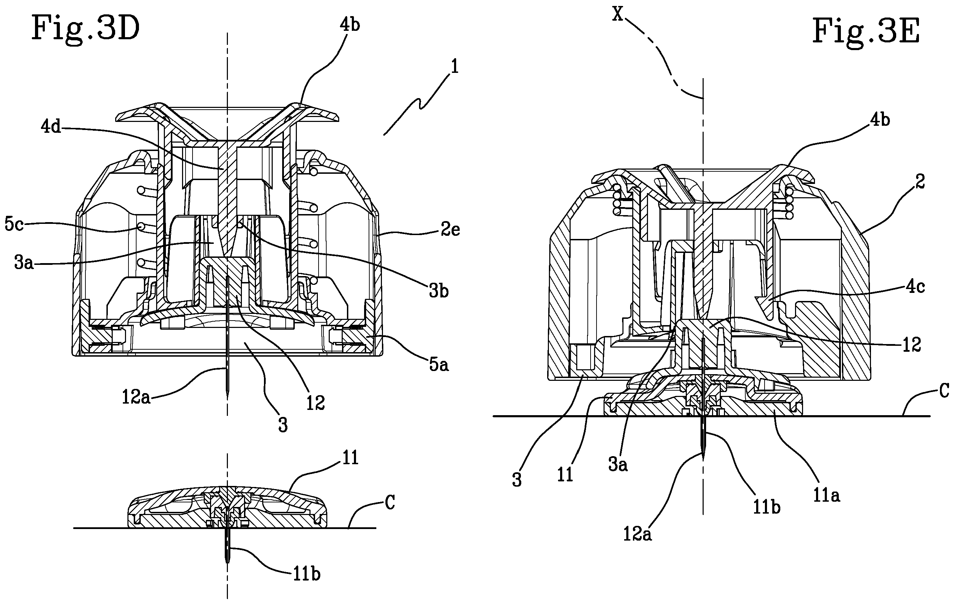

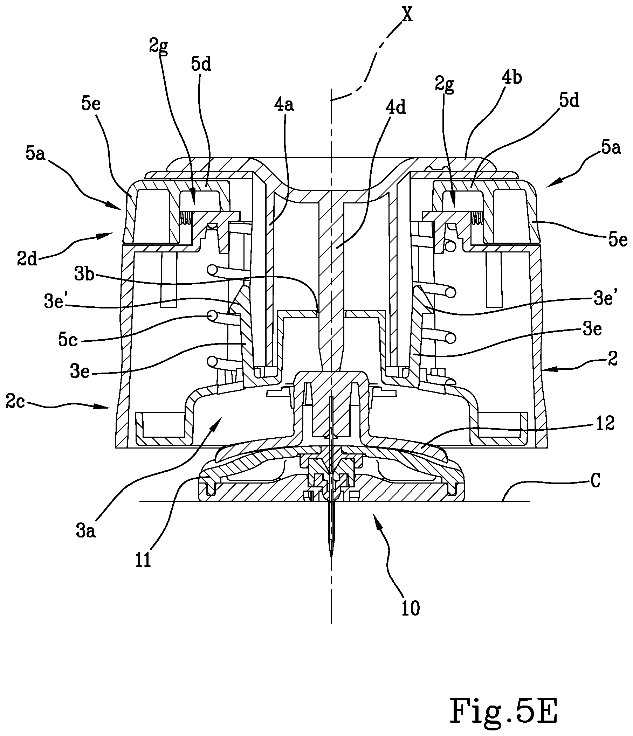

During this pressing of the dragging element 4 it is therefore possible to separate the infusion device 10 completely (Figures 3E and 5E) or at least partly (Figure 3D) from the containing seat 3a.

The separation of only the infusion body 11 or of the infusion body 11 and of the closing element 12 is performed as a function of the pressure exerted and/or the dimensional proportions between the containment seat 3a and the closing element 12.

In other words, once the dragging element 4 is pressed, there is the dual advantage of improving the adherence of the sticking plaster 11c to the skin "C" of the user and separating at least the infusion body 11 of the infusion device 10 from the containment seat 3a. In this way, once the applicator 1 has been moved away from the skin "C" of the user, at least the infusion body 11 of the infusion device 10 remains applied to the skin "C" of the patient without the risk that it can accidentally separate during this operation. Moreover, the improved adhesion of the sticking plaster 11c makes it possible to guarantee the adhesion of the infusion device 10 during the entire period necessary for the treatment to be performed. In other words, the improved adhesion of the sticking plaster 11c guarantees adhesion for extended periods of time of the infusion device 10 to the skin "C" of the patient.

The invention also relates to a method for using an infusion system 100 as described above.

The method comprises preparing an applicator 1 as described above and preparing an infusion device 10 as described above.

The method also comprises coupling the infusion device 10 by fitting in the containment seat 3a of the central body 3 as shown in Figures 3A and 5A. The method also comprises pressing the central body 3, to which the infusion device 10 is coupled, in the containment space defining the second configuration of the applicator 1 described above and positioning the applicator 1 on the skin "C" of the user, as shown in Figures 3B and 5B.

The method also comprises actuating the actuating means 5 moving the applicator 1 to the first configuration in such a way as to apply the infusion device 10 to the skin "C" of the patient as shown in Figures 3C, 5C and 5D.

Lastly, the method comprises pressing the dragging element 4 in such a way as to press the sticking plaster 11c of the infusion device 10 against the skin "C" of the patient and move the applicator 1 away from the skin "C" of the user (Figures 3D, 3E and 5E).

Preferably, the step of pressing the dragging element 4 comprises the separation at least of the infusion body 11 of the infusion device 10 from the containing seat 3a of the central body 3. In this way, during separation of the applicator 1 from the skin "C" of the user, the closing element 12 is dragged together with the applicator 1 allowing an immediate execution of the infusion treatment.

Preferably, during the step of pressing the dragging element 4 the closing element 12 is also separated from the containment seat 3a in such a way that, during the moving away of the applicator 1, the entire infusion device 10 remains applied to the skin "C" of the user.

Advantageously, the invention is able to overcome the drawbacks of the prior art.

Advantageously, the invention makes it possible to obtain an optimum adhesion of the infusion device 10 preventing the undesired detachment for long periods of adherence.

Advantageously, the invention is easy to use also for a non-expert user who simply by moving the applicator 1 close, actuating the actuating means 5 and pressing the dragging element 4 is able to easily obtain an effective and long-lasting application of the infusion device 10.

Moreover, the invention advantageously prevents detachment of the infusion device 10 during the moving away of the applicator 1 from the skin "C" of the user.

## Claims

1. An applicator (1) for infusion devices (10) comprising:
- a main body (2) comprising an access opening (2a) and an upper opening (2b) coaxial with each other and defined respectively in a lower portion (2c) and an upper portion (2d) of the main body (2);
- a central body (3) positioned inside said main body (2) and comprising a seat (3a) for containing said infusion device (10), said seat facing towards said access opening (2c) for housing said infusion device (10);
- a dragging element (4) comprising a first portion (4a), wherein a first end is connected or connectable to said central body (3) and passes through said upper opening (2d), and a second portion (4b), which can be pressed by a user, located at a second end of said first portion (4a),
- actuating means (5) connected or connectable to said central body (3);
said central body (3) being movable inside said main body (2) between a first configuration, wherein said central body (3) is positioned in said lower portion (2c) of the main body (2) and said first portion (4a) of the dragging element (4) is positioned inside the main body (2), and a second configuration wherein said central body (3) is positioned in said upper portion (2d) of the main body (2) and said first portion (4a) of the dragging element (4) is positioned outside the main body (2);
said central body (3) being movable by pressure by the user from the first configuration to the second and said actuating means (5) can be operated by the user for moving said central body (3) from the second to the first configuration for applying, in use, the infusion device (10), housed in the containment seat (3a) of the central body (3), to the skin (C) of a user;
**characterised in that** said second portion (4b) can be pressed, when said central body (3) is in said first configuration following the application of said infusion device (10), in such a way as to further move said central body (3) towards said access opening (2a) to promote a homogeneous adhesion of the infusion device (10) to the skin (C).

2. The applicator (1) according to claim 1, wherein said first portion (4a) of the dragging element comprises a plurality of flaps (4c) which can engage with a corresponding number of openings (3c) of the central member (3c).

3. The applicator (1) according to claim 1 or 2, wherein said containing seat (3a) of the central body (3) also comprises a through hole (3b) facing said dragging element (4) and wherein said dragging element (4) also comprises an elongate body (4d) configured for, during pressure of the second portion (4b), passing through said through hole (3b).

4. The applicator (1) according to any one of the preceding claims, wherein the containing seat (3a) of the central body (3) has a first portion directly facing the access opening (2a) which is substantially in the shape of a spherical cap and a second portion, located above said first portion, having a substantially cylindrical shape.

5. The applicator (1) according to any one of the preceding claims, wherein said main body (3) comprises protruding portions (3d) extending parallel to said first portion (4a) of the dragging element (4) and configured to define an end stop of said dragging element (4) when pressed.

6. The applicator (1) according to any one of the preceding claims, wherein said actuating means (5) comprise at least one button (5a) movable towards or away from said central body (3) and a loading spring (5c) whose ends are respectively connected to the central body (3) and to an internal perimeter edge of the upper opening (2d) of the main body (2).

7. The applicator (1) according to claim 6, wherein in said first configuration said loading spring (5c) extends between said internal perimeter edge and said central body (3) and said at least one button (5a) is positioned in contact with an inner wall of the main body (2),
wherein in said second configuration said loading spring (5c) is compressed between said internal perimeter edge and said central body (3) and said at least one button (5a) protrudes from a respective lateral opening (2e) of the main body (2) in such a way as to lock said central body (3) in said upper portion (2d), and
in said second configuration of the applicator (1), said at least one button (5a) being pressable in such a way as to release said central body (3) and move said applicator (1) to said first configuration for applying said infusion device (10).

8. The applicator (1) according to claim 6 or 7, wherein said central body (3) has a circular cross section and wherein said at least one button (5a) is movable radially relative to said central body (3).

9. The applicator (1) according to any one of claims 6 to 8, wherein said button (5a) is connected or connectable to said central body (3) by a suitable elastic element, preferably a spring (5b).

10. The applicator (1) according to any one of claims 1 to 6, wherein said main body (2) comprises, in said upper portion (2d), at least one guide (2g) having an end leading into a respective lateral opening (2e),
wherein said central body (3) comprises retaining portions (3e) extending parallel to said first portion (4a) of the dragging element (4), said retaining portions (3e) having a hook-shaped ends (3e') configured for engaging in an end of a respective guide (2g) which leads inside the main body (2), and
wherein said at least one button (5a) comprises a sliding portion (5d) configured to slide in the guide (2g) and a pressable portion (5e).

11. The applicator (1) according to claim 10, when dependent on claim 6, wherein in said first configuration said loading spring (5c) extends between said inner perimeter edge and said central body (3) and said end (3e') of the retaining portion (3e) is not engaged in the respective guide (2g);
wherein in said second configuration said loading spring (5c) is compressed between said inner perimeter edge and said central body (3) and said end (3e') of the retaining portion (3e) is engaged in the respective end of the guide (2g) in such a way as to lock said central body (3) in said upper portion (2d), and
in said second configuration of the applicator (1) said pressable portion (5e) of the at least one button (5a) being pressable in such a way as to make said sliding portion (5d) slide in said guide (5g) and move it towards said end (3e') of the retaining portion (3e) in such a way as to release said central body (3) and move said applicator (1) to said first configuration for applying said infusion device (10).

12. The applicator (1) according to claim 10 or 11, wherein said pressable portion (5e) is connected or connectable to said main body (2) by a suitable elastic element, preferably a spring (5b).

13. An infusion system (100) comprising:
- an applicator (1) according to any one of the preceding claims;
- an infusion device (10) comprising an infusion body (11), comprising a supporting base (11a) from which protrudes a cannula-shaped element (11b) of a catheter, and a removable closing element (12) equipped with a needle (12a) suitable for penetrating the skin (C) of the body of the user, said supporting base (11a) comprising a sticking plaster (11c) for allowing the attachment of the infusion device (10) to the body of the patient;
said infusion device (10) being designed to be coupled by fitting into said containment seat (3a) of the central body of the applicator (1).

14. The system (100) according to claim 13, wherein said containing seat (3a) is shaped to match said infusion device (10).

15. A method of preparing a system (100) according to claim 13 or 14 for attachment to the body of the patient, comprising the steps of:
- preparing an applicator (1) according to any one of claims 1 to 9;
- preparing an infusion device (10);
- coupling said infusion device (10) by fitting in said containment seat (3a) of the central body (3);
- pressing said central body (3), to which said infusion device (10) is coupled, in said main body (2) defining said second configuration;
- placing said applicator (1) on the skin (C) of a user;
- actuating said actuating means (5) moving said central body (3) to said first configuration in such a way as to apply said infusion device (10) to the skin (C) of the user;
- moving said central body (3) further towards said access opening (2a), by pressing the second portion (4a) of the dragging element (4), for promoting a homogeneous adhesion of the infusion device (10) to the skin (C);
- moving said applicator (1) away from the skin of the user (C).

16. The method of use according to claim 15, wherein said step of pressing the dragging element (4) comprises the separation at least of the infusion body (11) of the infusion device (10) from the containing seat (3a) of the central body (3), preferably said pressing step also comprising the separation of the closing element (12).

## Patentansprüche

1. Applikator (1) für Infusionsvorrichtungen (10), umfassend:
- einen Hauptkörper (2), der eine Zugangsöffnung (2a) und eine obere Öffnung (2b) umfasst, die koaxial zueinander sind und jeweils in einem unteren Abschnitt(2c) und einem oberen Abschnitt (2d) des Hauptkörpers (2) definiert sind;
- einen mittleren Körper (3), der im Inneren des Hauptkörpers (2) angeordnet ist und einen Sitz (3a) für die Aufnahme der Infusionsvorrichtung (10) umfasst, wobei der Sitz der Zugangsöffnung (2c) zugewandt ist, um die Infusionsvorrichtung (10) aufzunehmen;
- ein Mitnehmerelement (4), das einen ersten Abschnitt (4a) umfasst, wobei ein erstes Ende mit dem mittleren Körper (3) verbunden oder verbindbar ist und durch die obere Öffnung (2d) hindurchführt, und einen zweiten Abschnitt (4b) umfasst, der von einem Benutzer gedrückt werden kann und der an einem zweiten Ende des ersten Abschnitts (4a) angeordnet ist,
- Betätigungsmittel (5), die mit dem mittleren Körper (3) verbunden oder verbindbar sind;
wobei der mittlere Körper (3) innerhalb des Hauptkörpers (2) bewegbar ist zwischen einer ersten Konfiguration, in welcher der mittlere Körper (3) in dem unteren Abschnitt (2c) des Hauptkörpers (2) angeordnet ist und der erste Abschnitt (4a) des Mitnehmerelements (4) innerhalb des Hauptkörpers (2) angeordnet ist, und einer zweiten Konfiguration, in welcher der mittlere Körper (3) in dem oberen Abschnitt (2d) des Hauptkörpers (2) angeordnet ist und der erste Abschnitt (4a) des Mitnehmerelements (4) außerhalb des Hauptkörpers (2) angeordnet ist; wobei der mittlere Körper (3) durch die Druckausübung des Benutzers von der ersten Konfiguration in die zweite Konfiguration bewegbar ist und die Betätigungsmittel (5) durch den Benutzer betätigt werden können, um den mittleren Körper (3) von der zweiten Konfiguration in die erste Konfiguration zu bewegen, um im Gebrauch die Infusionsvorrichtung (10), die in dem Aufnahmesitz (3a) des mittleren Körpers (3) untergebracht ist, auf die Haut (C) eines Benutzers aufzubringen;
**dadurch gekennzeichnet, dass** der zweite Abschnitt (4b) gedrückt werden kann, wenn sich der mittlere Körper (3) nach dem Anbringen der Infusionsvorrichtung (10) in der ersten Konfiguration befindet, so dass der mittlere Körper (3) weiter in Richtung der Zugangsöffnung (2a) bewegt wird, um ein gleichförmiges Anhaften der Infusionsvorrichtung (10) an der Haut (C) zu begünstigen.

2. Applikator (1) nach Anspruch 1, wobei der erste Abschnitt (4a) des Mitnehmerelements eine Vielzahl von Laschen (4c) umfasst, die mit einer entsprechenden Anzahl von Öffnungen (3c) des mittleren Elements (3c) in Eingriff treten können.

3. Applikator (1) nach Anspruch 1 oder 2, wobei der Aufnahmesitz (3a) des mittleren Körpers (3) ferner ein Durchgangsloch (3b) umfasst, das dem Mitnehmerelement (4) zugewandt ist, und wobei das Mitnehmerelement (4) ferner einen länglichen Körper (4d) umfasst, der dafür ausgelegt ist, während der Druckausübung auf den zweiten Abschnitt (4b) durch das Durchgangsloch (3b) hindurchzutreten.

4. Applikator (1) nach einem beliebigen der vorhergehenden Ansprüche, wobei der Aufnahmesitz (3a) des mittleren Körpers (3) einen ersten Abschnitt aufweist, der direkt der Zugangsöffnung (2a) zugewandt ist und im Wesentlichen in Form einer kugelförmigen Kappe ausgebildet ist, und einen zweiten Abschnitt aufweist, der oberhalb des ersten Abschnitts angeordnet ist und eine im Wesentlichen zylindrische Form aufweist.

5. Applikator (1) nach einem beliebigen der vorhergehenden Ansprüche, wobei der Hauptkörper (3) hervorstehende Abschnitte (3d) aufweist, die sich parallel zu dem ersten Abschnitt (4a) des Mitnehmerelements (4) erstrecken und dafür ausgelegt sind, bei der Druckausübung einen Endanschlag für das Mitnehmerelement (4) zu definieren.

6. Applikator (1) nach einem beliebigen der vorhergehenden Ansprüche, wobei die Betätigungsmittel (5) zumindest einen Knopf (5a) umfassen, der in Richtung des mittleren Körpers (3) oder von diesem weg bewegbar ist, und eine Spannfeder (5c) umfassen, deren Enden jeweils mit dem mittleren Körper (3) und mit einem inneren Umfangsrand der oberen Öffnung (2d) des Hauptkörpers (2) verbunden sind.

7. Applikator (1) nach Anspruch 6, wobei sich in der ersten Konfiguration die Spannfeder (5c) zwischen dem inneren Umfangsrand und dem mittleren Körper (3) erstreckt und der zumindest eine Knopf (5a) in Kontakt mit einer inneren Wand des Hauptkörpers (2) angeordnet ist, wobei in der zweiten Konfiguration die Spannfeder (5c) zwischen dem inneren Umfangsrand und dem mittleren Körper (3) zusammengedrückt wird und der zumindest eine Knopf (5a) aus einer entsprechenden seitlichen Öffnung (2e) des Hauptkörpers (2) derart herausragt, dass der mittlere Körper (3) in dem oberen Abschnitt (2d) arretiert wird, und in der zweiten Konfiguration des Applikators (1) der zumindest eine Knopf (5a) derart gedrückt werden kann, dass der mittlere Körper (3) freigegeben und der Applikator (1) in die erste Konfiguration bewegt wird, um die Infusionsvorrichtung (10) anzubringen.

8. Applikator (1) nach Anspruch 6 oder 7, wobei der mittlere Körper (3) einen kreisförmigen Querschnitt aufweist und wobei der zumindest eine Knopf (5a) radial relativ zu dem mittleren Körper (3) bewegbar ist.

9. Applikator (1) nach einem der Ansprüche 6 bis 8, wobei der Knopf (5a) mittels eines geeigneten elastischen Elements, vorzugsweise einer Feder (5b), mit dem mittleren Körper (3) verbunden oder verbindbar ist.

10. Applikator (1) nach einem der Ansprüche 1 bis 6, wobei der Hauptkörper (2) in dem oberen Abschnitt (2d) zumindest eine Führung (2g) umfasst, die ein Ende aufweist, das in eine entsprechende seitliche Öffnung (2e) führt,
wobei der mittlere Körper (3) Rückhalteabschnitte (3e) umfasst, die sich parallel zu dem ersten Abschnitt (4a) des Mitnehmerelements (4) erstrecken, wobei die Rückhalteabschnitte (3e) hakenförmige Enden (3e') aufweisen, die dafür ausgelegt sind, mit einem Ende einer entsprechenden Führung (2g) in Eingriff zu gelangen, das in das Innere des Hauptkörpers (2) führt, und
wobei der zumindest eine Knopf (5a) einen Gleitabschnitt (5d) umfasst, der dafür ausgelegt ist, in der Führung (2g) zu gleiten, und einen drückbaren Abschnitt (5e) umfasst.

11. Applikator (1) nach Anspruch 10, sofern von Anspruch 6 abhängig, wobei sich in der ersten Konfiguration die Spannfeder (5c) zwischen dem inneren Umfangsrand und dem mittleren Körper (3) erstreckt und das Ende (3e') des Rückhalteabschnitts (3e) nicht mit der entsprechenden Führung (2g) in Eingriff steht,
wobei in der zweiten Konfiguration die Spannfeder (5c) zwischen dem inneren Umfangsrand und dem mittleren Körper (3) zusammengedrückt wird und das Ende (3e') des Rückhalteabschnitts (3e) derart mit dem entsprechenden Ende der Führung (2g) in Eingriff steht, dass der mittlere Körper (3) in dem oberen Abschnitt (2d) arretiert wird, und
in der zweiten Konfiguration des Applikators (1) der drückbare Abschnitt (5e) des zumindest einen Knopfes (5a) gedrückt werden kann, um zu bewirken, dass der Gleitabschnitt (5d) in der Führung (5g) gleitet und in Richtung des Endes (3e') des Rückhalteabschnitts (3e) bewegt wird, so dass der mittlere Körper (3) freigegeben wird und der Applikator (1) in die erste Konfiguration bewegt wird, um die Infusionsvorrichtung (10) anzubringen.

12. Applikator (1) nach Anspruch 10 oder 11, wobei der drückbare Abschnitt (5e) mittels eines geeigneten elastischen Elements, vorzugsweise einer Feder (5b), mit dem Hauptkörper (2) verbunden oder verbindbar ist.

13. Infusionssystem (100), umfassend:
- einen Applikator (1) nach einem beliebigen der vorhergehenden Ansprüche;
- eine Infusionsvorrichtung (10) mit einem Infusionskörper (11), umfassend eine Trägerbasis (11a), aus der ein kanülenförmiges Element (11b) eines Katheters herausragt, und ein abnehmbares Verschlusselement (12), das mit einer Nadel (12a) ausgestattet ist, die geeignet ist, in die Haut (C) des Körpers des Benutzers einzudringen, wobei die Trägerbasis (11a) ein Heftpflaster (11c) umfasst, welches das Anbringen der Infusionsvorrichtung (10) an den Körper des Patienten ermöglicht; wobei die Infusionsvorrichtung (10) dafür ausgelegt ist, durch Einpassen in den Aufnahmesitz (3a) des mittleren Körpers des Applikators (1) angekoppelt zu werden.

14. System (100) nach Anspruch 13, wobei der Aufnahmesitz (3a) so geformt ist, dass er der Infusionsvorrichtung (10) angepasst ist.

15. Verfahren zum Vorbereiten eines Systems (100) nach Anspruch 13 oder 14 zum Anbringen am Körper des Patienten, folgende Schritte umfassend:
- Vorbereiten eines Applikators (1) nach einem der Ansprüche 1 bis 9;
- Vorbereiten einer Infusionsvorrichtung (10);
- Ankoppeln der Infusionsvorrichtung (10) durch Einpassen in den Aufnahmesitz (3a) des mittleren Körpers (3);
- Eindrücken des mittleren Körpers (3), an den die Infusionsvorrichtung (10) gekoppelt ist, in den Hauptkörper (2), wodurch die zweite Konfiguration definiert wird;
- Aufsetzen des Applikators (1) auf die Haut (C) eines Benutzers;
- Betätigen der Betätigungsmittel (5) zum Bewegen des mittleren Körpers (3) in die erste Konfiguration, um die Infusionsvorrichtung (10) auf der Haut (C) des Benutzers anzubringen;
- Weiterbewegen des mittleren Körpers (3) in Richtung der Zugangsöffnung (2a) durch Drücken des zweiten Abschnitts (4a) des Mitnehmerelements (4), um ein gleichmäßiges Anhaften der Infusionsvorrichtung (10) an der Haut (C) zu begünstigen;
- Bewegen des Applikators (1) weg von der Haut des Benutzers (C).

16. Anwendungsverfahren nach Anspruch 15, wobei der Schritt des Drückens des Mitnehmerelements (4) die Trennung zumindest des Infusionskörpers (11) der Infusionsvorrichtung (10) von dem Aufnahmesitz (3a) des mittleren Körpers (3) umfasst, wobei der Schritt des Drückens vorzugsweise auch die Trennung des Verschlusselements (12) umfasst.

## Revendications

1. Un applicateur (1) pour dispositifs de perfusion (10) comprenant :
- un corps principal (2) comprenant une ouverture d'accès (2a) et une ouverture supérieure (2b) coaxiales l'une par rapport à l'autre et définies respectivement dans une portion inférieure (2c) et une portion supérieure (2d) du corps principal (2) ;
- un corps central (3) positionné à l'intérieur dudit corps principal (2) et comprenant un siège (3a) pour contenir ledit dispositif de perfusion (10), ledit siège étant orienté vers ladite ouverture d'accès (2c) pour loger ledit dispositif de perfusion (10) ;
- un élément d'entraînement(4) comprenant une première portion (4a), où une première extrémité est reliée ou peut être reliée audit corps central (3) et passe à travers ladite ouverture supérieure (2d), et une deuxième portion (4b), qui peut être pressée par un utilisateur, située au niveau d'une deuxième extrémité de ladite première portion (4a),
- des moyens d'actionnement(5) reliés ou pouvant être reliés audit corps central (3) ;
ledit corps central (3) étant mobile à l'intérieurduditcorps principal (2) entre une première configuration, dans laquelle ledit corps central (3) est positionné dans ladite portion inférieure (2c) du corps principal (2) et ladite première portion (4a) de l'élément d'entraînement (4) est positionnée à l'intérieur du corps principal (2), et une deuxième configuration dans laquelle ledit corps central (3) est positionné dans ladite portion supérieure (2d) du corps principal (2) et ladite première portion (4a) de l'élément d'entraînement (4) est positionnée à l'extérieur du corps principal (2) ;
ledit corps central (3) étant mobile par pression de la part de l'utilisateur de la première configuration à la deuxième et lesdits moyens d'actionnement (5) peuvent être actionnés par l'utilisateur pour déplacer ledit corps central (3) de la deuxième à la première configuration afin d'appliquer, lors de l'utilisation, le dispositif de perfusion (10), logé dans le siège-contenant (3a) du corps central (3), sur la peau (C) d'un utilisateur ;
**caractérisé en ce que** ladite deuxième portion (4b) peut être pressée, lorsque ledit corps central (3) est dans ladite première configuration suite à l'application dudit dispositif de perfusion (10), de manière à déplacer ultérieurement ledit corps central (3) vers ladite ouverture d'accès (2a) pour favoriser une adhésion homogène du dispositif de perfusion (10) à la peau (C).

2. L'applicateur (1) selon la revendication 1, dans lequel ladite première portion (4a) de l'élément d'entraînement comprend une pluralité d'ailettes (4c) qui peuvent s'assujettir avec un nombre correspondant d'ouvertures (3c) de l'organe central (3c).

3. L'applicateur (1) selon la revendication 1 ou 2, dans lequel ledit siège-contenant (3a) du corps central (3) comprend également un trou traversant (3b) face audit élément d'entraînement (4) et dans lequel ledit élément d'entraînement (4) comprend également un corps allongé (4d) configuré, lors d'une pression de la deuxième portion (4b), pour passer à travers ledit trou traversant (3b).

4. L'applicateur (1) selon l'une quelconque des revendications précédentes, dans lequel le siège-contenant (3a) du corps central (3) a une première portion, faisant directement face à l'ouverture d'accès (2a), qui a essentiellement la forme d'une calotte sphérique et une deuxième portion, située au-dessus de ladite première portion, qui a une forme essentiellement cylindrique.

5. L'applicateur (1) selon l'une quelconque des revendications précédentes, dans lequel ledit corps principal (3) comprend des portions en saillie (3d) s'étendant parallèlement à ladite première portion (4a) de l'élément d'entraînement (4) et configurées pour définir un fin de course dudit élément d'entraînement (4) lorsque pressé.

6. L'applicateur (1) selon l'une quelconque des revendications précédentes, dans lequel lesdits moyens d'actionnement(5) comprennent au moins un bouton (5a) mobile vers et à l'opposé dudit corps central (3) et un ressort de chargement (5c) dont les extrémités sont respectivement reliées au corps central (3) et à un bord périmétrique intérieur de l'ouverture supérieure (2d) du corps principal (2).

7. L'applicateur (1) selon la revendication 6, dans lequel, dans ladite première configuration, ledit ressort de chargement (5c) s'étend entre ledit bord périmétrique intérieur et ledit corps central (3) et ledit au moins un bouton (5a) est positionné en contact avec une paroi intérieure du corps principal (2),
dans lequel, dans ladite deuxième configuration, ledit ressort de chargement (5c) est compressé entre ledit bord périmétrique intérieur et ledit corps central (3) et ledit au moins un bouton (5a) dépasse d'une ouverture latérale (2e) respective du corps principal (2) de manière à bloquer ledit corps central (3) dans ladite portion supérieure (2d), et
dans ladite deuxième configuration de l'applicateur (1), ledit au moins un bouton (5a) pouvant être pressé de manière à libérer ledit corps central (3) et à déplacer ledit applicateur (1) dans ladite première configuration pour l'application dudit dispositif de perfusion (10).

8. L'applicateur (1) selon la revendication 6 ou 7, dans lequel ledit corps central (3) a une section circulaire et dans lequel ledit au moins un bouton (5a) est mobile radialement par rapport audit corps central (3).

9. L'applicateur (1) selon l'une quelconque des revendications de 6 à 8, dans lequel ledit bouton (5a) est relié ou peut être relié audit corps central (3) par un élément élastique approprié, de préférence un ressort (5b).

10. L'applicateur (1) selon l'une quelconque des revendications de 1 à 6, dans lequel ledit corps principal (2) comprend, dans ladite portion supérieure (2d), au moins un guide (2g) ayant une extrémité qui débouche dans une ouverture latérale (2e) respective,
dans lequel ledit corps central (3) comprend des portions de retenue (3e) s'étendant parallèlement à ladite première portion (4a) de l'élément d'entraînement (4), lesdites portions de retenue (3e) ayant des extrémités en forme de crochet (3e') configurées pour s'assujettir dans une extrémité d'un guide (2g) respectif qui débouche à l'intérieur du corps principal (2), et
dans lequel ledit au moins un bouton (5a) comprend une portioncoulissante (5d) configurée pour coulisser dans le guide (2g) et une portion pouvantêtre pressée (5e).

11. L'applicateur (1) selon la revendication 10, quand dépendant de la revendication 6, dans lequel, dans ladite première configuration, ledit ressort de chargement (5c) s'étend entre ledit bord périmétrique intérieur et ledit corps central (3) et ladite extrémité (3e') de la portion de retenue (3e) n'est pas assujettie dans le guide (2g) respectif ;
dans lequel, dans ladite deuxième configuration, ledit ressort de chargement (5c) est compressé entre ledit bord périmétrique intérieur et ledit corps central (3) et ladite extrémité (3e') de la portion de retenue (3e) est assujettie dans l'extrémité respective du guide (2g) de manière à bloquer ledit corps central (3) dans ladite portion supérieure (2d), et
dans ladite deuxième configuration de l'applicateur (1), ladite portion pouvant être pressée (5e) dudit au moins un bouton (5a) peut être pressée de manière à faire coulisser ladite portion coulissante (5d) dans ledit guide (5g) et à la déplacer vers ladite extrémité (3e') de la portion de retenue (3e) de manière à libérer ledit corps central (3) et à déplacer ledit applicateur (1) dans ladite première configuration pour l'application dudit dispositif de perfusion (10).

12. L'applicateur (1) selon la revendication 10 ou 11, dans lequel ladite portion pouvant être pressée (5e) est reliée ou peut être reliée audit corps principal (2) par un élément élastique approprié, de préférence un ressort (5b).

13. Un système de perfusion (100) comprenant :
- un applicateur (1) selon l'une quelconque des revendications précédentes ;
- un dispositif de perfusion (10) comprenant un corps de perfusion (11), comprenant une base de support (11a) de laquelle dépasse un élément en forme de canule (11b) d'un cathéter, et un élément de fermeture (12) amovible muni d'une aiguille (12a) conçue pour pénétrer dans la peau (C) du corps de l'utilisateur, ladite base de support (11a) comprenant un sparadrap (11c) pour permettre la fixation du dispositif de perfusion (10) sur le corps du patient ;
ledit dispositif de perfusion (10) étant destiné à être accouplé par emboîtement dans ledit siège-contenant (3a) du corps central de l'applicateur (1).

14. Le système (100) selon la revendication 13, dans lequel ledit siège-contenant (3a) est conformé pour correspondre à la forme dudit dispositif de perfusion (10).

15. Un procédé de préparation d'un système (100) selon la revendication 13 ou 14 pour la fixation au corps du patient, comprenant les étapes consistant à :
- préparer un applicateur (1) selon l'une quelconque des revendications de 1 à 9 ;
- préparer un dispositif de perfusion (10) ;
- accoupler ledit dispositif de perfusion (10) en l'emboîtant dans ledit siège-contenant (3a) du corps central (3) ;
- presser ledit corps central (3), auquel ledit dispositif de perfusion (10) est accouplé, dans ledit corps principal (2) définissant ainsi ladite deuxième configuration ;
- placer ledit applicateur (1) sur la peau (C) d'un utilisateur ;
- actionner lesdits moyens d'actionnement (5) en déplaçant ainsi ledit corps central (3) dans ladite première configuration de manière à appliquer ledit dispositif de perfusion (10) sur la peau (C) de l'utilisateur ;
- déplacer encore ledit corps central (3) vers ladite ouverture d'accès (2a), en pressant la deuxième portion (4a) de l'élément d'entraînement (4), pour favoriser une adhésion homogène du dispositif de perfusion (10) à la peau (C) ;
- éloigner ledit applicateur (1) de la peau de l'utilisateur (C).

16. Le procédé d'utilisation selon la revendication 15, dans lequel ladite étape consistant à presser l'élément d'entraînement (4) comprend la séparation au moins du corps de perfusion (11) du dispositif de perfusion (10) du siège-contenant(3a) du corps central (3), de préférence ladite étape de pression comprenant également la séparation de l'élément de fermeture (12).
